# EUROPEAN PATENT SPECIFICATION

(11) **EP 3 962 929 B1**
(45) Date of publication and mention of the grant of the patent: **01.03.2023**
(21) Application number: 20725940.9
(22) Date of filing: 24.04.2020
(51) Int. Cl.: C07K 9/00, C07K 1/20

(54) **METHOD FOR THE PURIFICATION OF LIPOGLYCOPEPTIDE ANTIBIOTICS**
VERFAHREN ZUR REINIGUNG VON LIPOGLYKOPEPTID-ANTIBIOTIKA
PROCÉDÉ DE PURIFICATION D'ANTIBIOTIQUES LIPOGLYCOPEPTIDIQUES

(30) Priority: 30.04.2019 IT 201900006442
(43) Date of publication of application: 09.03.2022
(73) Proprietor: Huvepharma Italia S.r.l., 12075 Garessio (CN) (IT)
(72) Inventor: CAPOZZOLI, Carmine, 21040 Gerenzano (Varese) (IT); TERRANEO, Alberto, 21040 Gerenzano (Varese) (IT)
(74) Representative: Cesa, Roberta
(86) International application number: PCT/IB2020/053884
(87) International publication number: WO 2020/222100

(56) References cited:
- CN-A- 110 412 142
- US-A- 4 782 042
- US-A1- 2017 190 744
- XIAOTONG ZHANG ET AL: "Evaluation of dalbavancin as chiral selector for HPLC and comparison with teicoplanin-based chiral stationary phases", CHIRALITY., 1 January 2009 (2009-01-01), pages 495-513, XP055649791, US ISSN: 0899-0042, DOI: 10.1002/chir.20771

## Description

### Field of the invention

The present description relates to methods for purifying active principles. More specifically, the description relates to methods for purifying lipoglycopeptide antibiotics.

### Background

Dalbavancin is included among the lipoglycopeptide antibiotics known today. Dalbavancin is a second generation semisynthetic lipoglycopeptide with activity towards a broad spectrum of Gram-positive pathogenic microorganisms. The use of dalbavancin for treating Gram-positive bacterial infections is, for example, described in documents US 6,900,175 B2, US 2005/0004050 A1 and US 2009/0298749 A1.

The semi-synthesis of the dalbavancin molecule starting from the precursor of natural origin A40926 - obtained by fermentation carried out by the microorganism *Nonomuraea gerenzanensis* - is described, for example, in the document US 6,900,175 B2.

Methods known to date for purifying the unrefined (crude) compound A-40926 and dalbavancin are carried out by adsorption chromatography on polyamide. These methods, however, can present critical issues due to sub-optimal operating conditions, and the obtaining of a final product that may contain impurities. In particular, the choice of using adsorption chromatography on polyamide may require a complex fractionation work with a step gradient using carbonate/bicarbonate buffered solutions. Furthermore, these operating conditions can lead to the formation of an isomerization impurity that is difficult to control.

US 2017/190744 discloses a method for purifying dalbavancin as a lipopeptide antibiotic.

US 4 782 042 discloses a method for purifying precursor compounds of the antibiotic A40926. The method, however, comprises a step of concentration of the eluate fractions carried out under vacuum followed by a phase of elimination of the inorganic salts by means of a silica gel column. The eluate fractions are then further concentrated under vacuum.

There is, therefore, the need to provide complex and repeated fractionation steps which may have the disadvantage of reducing the yield of the method in relation to obtaining a product whose purity can be increased.

### Summary of the invention

The present description is intended to provide purifying methods for lipoglycopeptide antibiotics that are simple, with reduced environmental impact and capable of providing high purity products.

According to the present description, the above object is achieved thanks to the subject to which specific reference is made in the following claims, intended as an integral part of the present description.

One embodiment of the present description provides a method for purifying at least one lipoglycopeptide antibiotic comprising the steps of:
i) dissolving said at least one lipoglycopeptide antibiotic in an aqueous solution to form a mixture,
ii) loading said mixture into a chromatographic column comprising a stationary phase, wherein said stationary phase comprises silica functionalized with organic pendants,
iii) eluting the mixture loaded in step ii) using an eluent composition comprising a water-soluble organic solvent obtaining eluate fractions,
iv) selecting the eluate fractions containing the at least one purified lipoglycopeptide antibiotic.

In one or more embodiments, the silica functionalized with organic pendants may comprise octadecyl silyl derivatized silica, octyl silyl derivatized silica, exylphenyl silyl derivatized silica, or butyl silyl derivatized silica.

The silica functionalized with organic pendants of the stationary phase may have a particle size of less than 50 µm, preferably less than 20 um.

The stationary phase may also be conditioned prior to the loading step ii) with a mobile phase that comprises at least one compound selected in the group consisting of ammonium formate, ammonium acetate or triethylamine salts with formic acid, acetic acid, trifluoroacetic acid. This mobile phase may have a pH between 5.0 and 7.0.

The method according to the invention comprises the steps of:
v) concentrating the eluate fractions collected in step iv) to obtain a concentrate,
vi) optionally dialyzing the concentrate obtained in step v),
vii) precipitating said concentrate, optionally dialyzed in step vi), with a precipitation solution comprising at least one organic solvent to obtain a precipitate,
viii) drying the precipitate obtained in step vii), wherein said concentration step v) is carried out by means of a membrane nanofiltration system with a molecular cut-off ranging from 100 Da to 1500 Da.

### Brief description of the drawings

The invention will now be described, by way of example, with reference to the attached figures, wherein:
- Figure 1 illustrates the chromatographic profile of the purification of A40926 conducted according to embodiments of the present description,
- Figure 2 illustrates the chromatographic profile of the purification of dalbavancin conducted according to embodiments of the present description,
- Figure 3 illustrates the chromatographic profile of the purification of dalbavancin conducted according to embodiments of the present description,
- Figure 4 illustrates the chromatographic profile of the purification of dalbavancin conducted according to embodiments of the present description,
- Figure 5 illustrates the analytical HPLC profile of dalbavancin currently commercially available,
- Figure 6 illustrates the analytical HPLC profile of purified dalbavancin according to embodiments of the present description.

### Detailed description of preferred embodiments

In the following description, numerous specific details are provided to allow a thorough understanding of embodiments. The embodiments can be implemented without one or more of the specific details or with other methods, components, materials etc. In other cases, well-known structures, materials or operations are not shown or described in detail to avoid confusing aspects of the embodiments.

Reference throughout the present disclosure to "one embodiment" or "an embodiment" indicates that a particular aspect, structure or characteristic described with reference to the embodiment is included in at least one embodiment. Thus, forms of the expressions "in one embodiment" or "in an embodiment" at various points throughout the present description do not necessarily all refer to the same embodiment. Moreover, the particular aspects, structures or characteristics can be combined in any convenient way in one or more embodiments. The titles provided in this description are for convenience only and do not interpret the scope or object of the embodiments.

The Inventors of this application have identified specific operating conditions that favor the obtainment of effective purifications of lipoglycopeptide antibiotics by means of a simple method, characterized by lower costs and reduced environmental impact compared to the lipoglycopopeptide antibiotic purification methods known in the art.

One embodiment of the present description provides a method for purifying at least one lipoglycopeptide antibiotic comprising the steps of:
i) dissolving said at least one lipoglycopeptide antibiotic in an aqueous solution to form a mixture,
ii) loading said mixture into a chromatographic column comprising a stationary phase, wherein said stationary phase comprises silica functionalized with organic pendants,
iii) eluting the mixture loaded in said step ii) using an eluent composition comprising a water-soluble organic solvent obtaining eluate fractions,
iv) selecting the eluate fractions containing the at least one purified lipoglycopeptide antibiotic.

In one or more embodiments, the selected eluate fractions have a purity equal to or greater than 90%.

Lipoglycopeptide antibiotics that can be purified using the method described in the present description may, for example, be selected in the group consisting of A40926, dalbavancin, teicoplanin, mideplanin (MDL-62873), and ramoplanin.

Dalbavancin and the compound A40926 (its intermediate) to be subjected to the method described in the present description can be obtained by methods known in the art. A40926 is a fermentation product which can be isolated, for example, from a fermentation broth of *Nonomuraea gerenzanensis* ATCC 39727 by filtration, affinity chromatography and concentration by azeotropic distillation with butanol, followed by the addition of petroleum ether to precipitate the unrefined product as, for example, described in the document US 4,935,238.

Dalbavancin can be obtained, for example, by amidation of the monomethyl ester of A40926 and subsequent hydrolysis, as described in the document US 2004/0142883 A1.

According to the purification method described here, the antibiotic to be subjected to purification is dissolved in an aqueous solution to obtain a mixture.

In one or more embodiments, said mixture may comprise the at least one antibiotic to be subjected to purification in a concentration of between 25 g/l and 45 g/l.

In one embodiment, step i) may comprise adding a component selected in the group consisting of formic acid, sulfuric acid, acetic acid, trifluoroacetic acid, hydrochloric acid, and phosphoric acid, preferably hydrochloric acid, to the aqueous solution. For example, the method may comprise the step of adding an acid component selected in the group consisting of formic acid, sulfuric acid, acetic acid, trifluoroacetic acid, hydrochloric acid, and phosphoric acid to the aqueous solution, when the antibiotic to be purified is dalbavancin, or teicoplanin, or mideplanin (MDL-62873), or ramoplanin.

In another embodiment, step i) may comprise adding a component selected in the group consisting of ammonia, sodium hydroxide, potassium hydroxide, sodium carbonate, potassium carbonate, sodium bicarbonate, and potassium bicarbonate, preferably sodium hydroxide. For example, the method may comprise the step of adding a basic component to the aqueous solution selected in the group consisting of ammonia, sodium hydroxide, potassium hydroxide, sodium carbonate, potassium carbonate, sodium bicarbonate, and potassium bicarbonate, preferably sodium hydroxide when the antibiotic to be purified is A40926.

The mixture thus obtained is loaded onto a chromatographic column with a stationary phase which may comprise silica functionalized with organic pendants.

The expression silica functionalized with organic pendants means silica to which aliphatic or aryl aliphatic organic chains have been covalently linked, through siloxane bonds.

The silica functionalized with organic pendants may be selected in the group consisting of octadecyl silyl derivatized silica, octyl silyl derivatized silica, exylphenyl silyl derivatized silica, and butyl silyl derivatized silica.

Advantageously, the ratio between the quantity of the loaded mixture and the volume of the stationary phase is between 0.1 g/l and 1.6 g/l.

The inventors of the present application have observed that particularly advantageous results are obtained when the stationary phase comprises octadecyl silyl derivatized silica (C18), preferably with a particle size lower than 50 µm, more preferably lower than 20 um.

The stationary phase can be conditioned, prior to the loading step ii), with a mobile phase which may have a pH between 5.0 and 7.0.

For conditioning the stationary phase, this mobile phase may comprise an aqueous solution of at least one compound selected in the group consisting of ammonium formate, ammonium acetate or an aqueous solution comprising triethylamine salts with formic acid, acetic acid, and trifluoroacetic acid.

In one or more embodiments, the at least one compound of the mobile phase has a concentration of between 0.01% and 10% w/v of the aqueous solution.

In one or more embodiments, the mobile phase comprises an aqueous solution of ammonium formate, preferably in a molar concentration of between 0.0025M and 0.05M.

In one or more embodiments, the mobile phase may comprise an aqueous solution of triethylamine and formic acid, preferably each in a concentration comprised between 0.005% w/w and 0.2% w/w.

After loading the mixture comprising the antibiotic to be subjected to purification, the elution step follows with an eluent composition, which may comprise at least one water-soluble organic solvent selected in the group consisting of methanol, propanol, isopropanol, acetonitrile, and acetone. Preferably, the eluent composition comprises acetonitrile.

The concentration of the water-soluble organic solvent can be between 10% and 90% v/v of the eluent composition.

In one or more embodiments, the eluent composition may comprise at least one additional compound selected in the group consisting of triethylamine, formic acid, acetic acid, trifluoroacetic acid, heptafluorobutyric acid, methanesulfonic acid, ammonium formate, and ammonium acetate. This additional component can be present in a concentration comprised between 0.005% w/w and 0.2% w/w.

The elution step iii) can be carried out at a pH between 5.0 and 7.0, preferably between 5.2 and 6.5.

The elution step may be carried out at a flow of between 3 and 5 bed volumes/hour.

In one or more embodiments, the eluent composition may comprise said water-soluble organic solvent, preferably acetonitrile, in a concentration increasing over time, at least in one interval of conduction time of the elution step iii) or along the entire elution step iii). The concentration, for example, of acetonitrile, may increase from a minimum value of 10% to a maximum value of 90% v/v of the eluent composition in said at least one interval of conduction time of the elution step iii) or along the entire elution step iii). The eluent composition comprising said at least one organic solvent in increasing concentration may be obtained by mixing i) a pure solution of said at least one organic solvent, for example, acetonitrile, with ii) an aqueous solution. The mixing can be carried out using a pure solution quantitative ratio of said at least one organic solvent:aqueous solution variable over the conduction time of the eluent step. This quantitative ratio may vary, for example, between a minimum value of 0.1 (pure solution: aqueous solution ratio 10:90) to a maximum value of 9 (pure solution:diluent solution 90:10). The aqueous solution may comprise at least one compound selected in the group consisting of triethylamine, formic acid, acetic acid, trifluoroacetic acid, heptafluorobutyric acid, methanesulfonic acid, ammonium formate, and ammonium acetate, preferably in a concentration of between 0.005% w/w and 0.2% w/w.

The method may comprise a step of selecting the eluate fractions and collecting the selected eluate fractions. Selecting eluate fractions is based on the degree of purity of the antibiotic subjected to the method in question. Preferably, the method envisages selecting eluate fractions with a purity greater than or equal to 90% (greater than or equal to 90% considering the sum of the compounds of interest contained in the mixture).

The selection of the eluate fractions to be concentrated is carried out by HPLC analysis using, for example, the method reported in the document US 6,900,175 B2, or official pharmacopoeial compendial methods.

The selected and collected eluate fractions can consequently be subjected to a concentration step to obtain a concentrate.

This concentration step of the fractions is carried out using a membrane nanofiltration system with a molecular cut-off ranging from 100 Da and 1500 Da.

The concentration step of the fractions allows a quantity of purified antibiotic to be obtained in the concentrate ranging from 10 mg/ml and 150 mg/ml, preferably from 20 mg/ml to 80 mg/ml.

The method may also comprise a step of adjusting the pH of the concentrate to a value from 2.5 to 4.0 by adding, in the concentrate, an aqueous solution of an acid, for example, hydrochloric acid. In one or more embodiments, the aqueous solution of an acid may comprise 15% w/w of hydrochloric acid.

The excess water in the concentrate can be eliminated, for example, by means of nanofiltration or reverse osmosis.

In one or more embodiments, the method may include a dialyzing step of the concentrate. This step allows elimination from the concentrate of the at least one compound contained in the mobile phase used for conditioning. Furthermore, this dialyzation step may allow removal of traces of the at least one organic solvent and/or of the possible additional compound selected in the group consisting of triethylamine, formic acid, acetic acid, trifluoroacetic acid, heptafluorobutyric acid, methanesulfonic acid, ammonium formate, and ammonium acetate used in the eluent composition of the elution step.

In one or more embodiments, the method may also comprise at least one step of precipitating the concentrate with a precipitation solution comprising at least one organic solvent selected in the group consisting of acetonitrile, acetone, isopropanol, propanol and ethanol, preferably acetone. The concentrate can be resuspended in water and then in acetone to obtain the complete precipitation of the required product.

In the precipitation step, the pH can be adjusted to a value between 4.0 and 6.0, preferably 5, with an alkaline aqueous solution, for example, of sodium hydroxide. For example, the alkaline solution may comprise 20% w/w sodium hydroxide.

The precipitate obtained can be filtered and washed with an organic solvent, for example acetone.

A step follows in which the precipitate is dried, for example, under vacuum at a temperature of 30°C.

In one or more embodiments, the precipitate may be freeze-dried, for example, at a temperature of 15°C and a pressure of 50 microbar.

The described method, compared to methods known in the art, has a number of advantages. For example, the choice of the specific stationary phase and of an eluent comprising a water soluble organic solvent allows a more effective purification to be obtained. The method, in fact, allows elimination of colored compounds, less polar impurities of the antibiotic of interest and - at the same time - more polar ones, which would otherwise require separate purification steps.

In addition, the concentration step of the selected eluate fractions is simple and fast thanks to the use of nanofiltration; the choice of this operating condition in combination with controlling the working pH allows avoiding the degradation of the products obtained, degradation that can occur, for example, when the purification method involves concentration steps obtained by hot distillation.

The described method allows a high yield to be obtained which is between 80.0% and 96.0%.

In addition, as will be evident below, the purity of the product obtained is significantly higher than the purity of products available on the market and obtained with the methods known in the art.

### EXAMPLES

### Example 1 - Purification method of the compound A40926

On a preparative HPLC chromatographic column of 10 cm diameter and 60 cm packed height with stationary phase compound of octadecyl silyl derivatized silica (C18) with a particle size of 15 um (Luna C18, Phenomenex), conditioned with a mobile phase comprising 0.025 M aqueous pH 6.5 ammonium formate (CarloErba) (mobile phase), 200 ml of an aqueous solution of 20 g of unrefined A40926 (4.9 g antibiotic activity) was loaded.

Unrefined A-40926 was obtained by submerged fermentation of *N. gerenzanensis,* as described in the document US 4,935,238, followed by microfiltration of the harvest broth, concentration by nanofiltration and precipitation of the unrefined product, as described in the document US 6,900,175 B2. In particular, the method for obtaining unrefined A-40926 comprises the steps described below. A cryotube of the *N. gerenzanensis* strain producing A40926 is used to inoculate a 500 mL (Erlenmeyer) flask containing 50 mL of culture medium A composed as follows: 1 g/l Dextrose (Roquette); 24 g/l Soluble starch (Difco-BD); 5 g/l Yeast extract (Constantine); 5 g/l Tryptose (Difco-BD); 4 g/l calcium carbonate (Imerys). The culture is incubated at 28°C on a 240 rpm rotary shaker.

After 72 h, 10% of the culture is transferred to a 500 mL (Erlenmeyer) flask containing 100 ml of culture medium B with the following composition: 25 g/l Dextrose (Roquette); 4 g/l Yeast autolysate (Constantine); 20 g/l Soybean meal (Mucedola); 1.25 g 1 sodium chloride (Carlo Erba); 5 g/l calcium carbonate (Imerys); 0.6 g/l Defoamer (Momentive Performance Materials Inc); (pH 7.6 before sterilization).

After 96 hours, 3% of the culture is transferred to a 20 1 prefermenter containing 16 1 of culture medium B. The fermenter is stirred at about 900 rpm with a sterile air flow equal to 4 liters per minute. After 72 hours, the content is transferred to a 200 1 fermenter containing 145 1 of culture medium B. After 168 hours, the maximum production of A40926 and its acetylated derivative is reached (about 1 g/l of A40926).

The culture broth is treated with 20% sodium hydroxide up to pH 11.4, keeping the temperature at 23°C. It is kept at this temperature for 6 hours, then it is cooled to 15°C and it is subjected to microfiltration on ceramic membranes (Koch, 0.1 micron). The permeate, containing A40926, is brought to pH 8.5 with 15% hydrochloric acid and subjected to concentration by means of nanofiltration (membranes 250 Da, Koch) until a solution with a concentration of 40 g/l of A40926 is obtained. The solution is then treated with 9 volumes of acetone obtaining the precipitation of unrefined A40926, which is then dried under vacuum to a water content of less than 10%.

The column was then eluted at 320 ml/min with an eluent composition comprising acetonitrile (CarloErba) in an increasing concentration over the elution conduction time. This eluent composition was obtained following the mixing in time-varying quantitative ratios, as illustrated in Table 1, of a first solution (mobile phase A) comprising demineralized water with the addition of 0.025M ammonium formate (CarloErba) at pH 6.5, and a second solution (mobile phase B) of pure acetonitrile (CarloErba).

**Table 1**

| **Time** | **Mobile phase A** | **Mobile phase B** |
|---|---|---|
| **(min)** | % **(v/v)** | % **(v/v)** |
| **0** | 80 | 20 |
| **5** | 75 | 25 |
| **45** | 69 | 31 |
| **54** | 69 | 31 |
| **55** | 10 | 90 |
| **70** | 10 | 90 |

The eluate was collected in 4 fractions as indicated in Table 2.

**Table 2**

| **Fraction** | **Collection (min)** | **Concentration** | **Fraction weight** |
|---|---|---|---|
| **F1** | 32 - 36.5 | 0.049 g/l | 2615 g |
| **F2** | 36.5 - 38.6 | 0.121 g/l | 2000 g |
| **F3** | 38.6 - 46.3 | 1.864 g/l | 3530 g |
| **F4** | 46.3 - 49.7 | 0.462 g/l | 2305 g |

The yield of the purification method was 93%.

The fractions containing A40926 that resulted in the specification were concentrated and dialyzed against demineralized water (10 diavolumes) with a nanofiltration system with a molecular cut-off of 250 Da (HydroAirResearch) until a concentration of A40926 in the concentrate of 20 g/l was obtained. Dialysis allows removal of the ammonium formate present in the eluent mixture.

The selection of the eluate fractions to be concentrated is carried out by HPLC analysis by choosing the fractions with purity greater than or equal to 90%, calculated on the sum of the areas of the peaks of the antibiotic subjected to purification.

The concentrate was brought to pH 3.8 +/- 0.2 with 15% w/w aqueous hydrochloric acid (CarloErba) and centrifuged to remove the water. The solid residue was resuspended with 20 volumes of water with respect to A40926 followed by 20 volumes of acetone (CarloErba), and the pH raised to 5 with 20% aqueous sodium hydroxide (CarloErba). Another 120 volumes of acetone (CarloErba) were then added, obtaining the complete precipitation of the required product. The solid was recovered by filtration, washed with 20 volumes of acetone (CarloErba) and dried under vacuum at 30°C. The yield of the process was 92.4%.

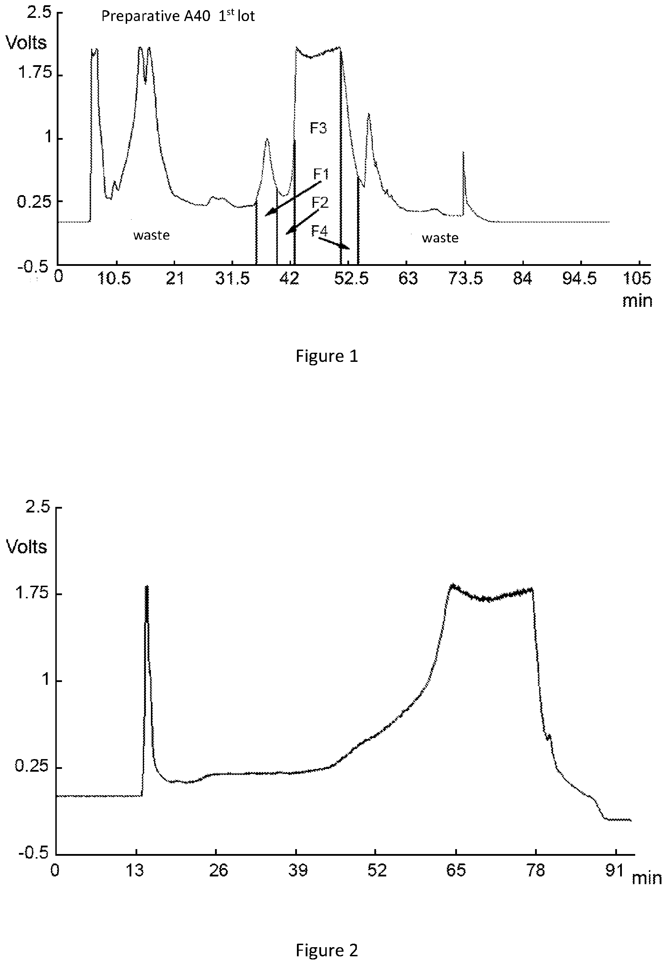

Figure 1 illustrates the chromatographic profile (tracing of the UV signal read at 280 nm) of the purification of A40926 obtained by preparative HPLC carried out by applying the conditions of example 1. The vertical lines indicate the boundary between one collection fraction and the next. As can be seen immediately, the fractions F1 and F3 correspond to two main peaks of the chromatogram: they contain the active components of the antibiotic A40926. The impurities are eliminated in the fractions called "waste" and F2 and F4.

### Example 2 - Purification method of dalbavancin

On a preparative HPLC chromatographic column of 10 cm diameter and 60 cm packed height with stationary phase compound of octadecyl silyl derivatized silica (C18) with a particle size of 15 um (Phenomenex), conditioned with a mobile phase of 0.025 M aqueous pH 6.5 ammonium formate (CarloErba) (mobile phase), 680 ml of an aqueous solution of unrefined dalbavancin (7.3 g) was loaded.

Dalbavancin was obtained from A40926 through a sequence of reactions that provide protection such as monomethyl ester, amidation with 3-N,N-dimethylaminopropylamine final deprotection to give Dalbavancin as described in document US 6,900,175 B2.

In particular, the method of obtaining dalbavancin comprises the steps of: methylation of A40926 in methanol (CarloErba) catalyzed by concentrated sulfuric acid (CarloErba) followed by precipitation of the methyl ester of A40926 by dilution with water and pH correction between 4.5 and 6.5 with triethylamine (CarloErba). Amidation of methyl ester into dimethyl sulfoxide (CarloErba) and methanol (CarloErba) with 3-N,N-dimethylaminopropylamine (SigmaAldrich) using dicyclohexylcarbodiimide (SigmaAldrich) as the condensing agent. The methyl ester amide obtained is hydrolyzed with 20% aqueous sodium hydroxide (CarloErba) to give an unrefined dalbavancin solution which, after correcting the pH to 3.5 with 15% aqueous hydrochloric acid (CarloErba), is used for the purification tests as such, or by preceding a dialysis against water to remove the organic solvents present (dimethyl sulfoxide and methanol).

The column was then eluted at 320 ml/min with an eluent composition comprising acetonitrile (CarloErba) in an increasing concentration over the elution conduction time. This eluent composition was obtained following the mixing, in time-varying quantitative ratios, as illustrated in Table 3, of a first solution (mobile phase A) comprising demineralized water with the addition of 0.025M aqueous ammonium formate (CarloErba) at pH 6.5, and a second solution (mobile phase B) of pure acetonitrile (CarloErba).

**Table 3**

| **Time** | **Mobile phase A** | **Mobile phase B** |
|---|---|---|
| **(min)** | **% (v/v)** | % **(v/v)** |
| **0** | 80 | 20 |
| **5** | 80 | 20 |
| **40** | 65 | 35 |
| **50** | 60 | 40 |
| **60** | 55 | 45 |
| **70** | 55 | 45 |
| **71** | 10 | 90 |
| **85** | 10 | 90 |

The eluate was collected in 7 fractions as described in Table 4.

**Table 4**

| **Fraction** | **Collection** | **Concentration** | **Fraction volume** |
|---|---|---|---|
| | **(min)** | **(g/1)** | **(1)** |
| **F1** | 20-37 | - | 5.4 |
| **F2** | 37-49.6 | 0.1 | 4.0 |
| **F3** | 49.6-56 | 0.1 | 2.0 |
| **F4** | 56-69 | 0.4 | 4.2 |
| **F5** | 69-78 | 0.8 | 2.9 |
| **F6** | 78-85 | 1.0 | 2.2 |
| **F7** | 85-93 | 0.3 | 2.6 |

The yield of the purification method was found to be 95%.

The fractions containing dalbavancin, resulting in specification, were concentrated and dialyzed against demineralized water (10 diavolumes), with a nanofiltration system with a molecular cut-off of 250 Da (HydroAirResearch) until a concentration of dalbavancin in the concentrate of 80 g/l was obtained. The selection of the eluate fractions to be concentrated is carried out by HPLC analysis by choosing the fractions with purity greater than or equal to 90% calculated on the sum of the areas of the peaks of the compounds of interest.

The concentrate was then brought to pH 2.6 +/- 0.1 with 15% w/w aqueous hydrochloric acid (Carlo Erba). A total of 9 volumes of acetone (CarloErba) were then added to the dalbavancin solution in water at a temperature below 10°C. The solid is recovered after overnight incubation at 4°C by filtration on a porous septum, washed with 1.5 volumes of acetone (CarloErba) and dried under vacuum (50 ubar) at 15°C. The yield of the method was 84.0%.

Figure 2 illustrates the chromatographic profile (tracing of the UV signal read at 280 nm) of the purification of dalbavancin obtained by preparative HPLC carried out by applying the conditions of example 2.

The apparent absence of resolution is due to the extreme concentrations of the material that saturates the detector of the instrument. However, it is possible to note that in the initial and final parts of the chromatogram there are peaks, which testify successful separation of the impurities.

### Example 3 - Purification of dalbavancin

On a preparative HPLC chromatographic column of 10 cm diameter and 60 cm packed height with stationary phase compound of octadecyl silyl derivatized silica (C18) with a particle size of 15 um (Phenomenex), conditioned with an aqueous solution of 0.1% v/v triethylamine (CarloErba) and 0.1% v/v formic acid (CarloErba) (mobile phase A), 680 ml of an aqueous solution of unrefined dalbavancin (7.3 g active) was loaded, obtained as described in Example 2.

The column was then eluted at 320 ml/min with an eluent composition comprising acetonitrile (CarloErba) in an increasing concentration over the elution conduction time. This eluent composition was obtained following the mixing, in quantitative ratios varying over time, as illustrated in Table 5, of a first solution (mobile phase A) comprising demineralized water with the addition of 0.1% v/v triethylamine (CarloErba) and 0.1% v/v formic acid (CarloErba) and a second solution (mobile phase B) of pure acetonitrile (CarloErba) supplemented with 0.1% v/v triethylamine (CarloErba) and 0.1% v/v formic acid (CarloErba).

**Table 5**

| **Time** | **Mobile phase A** | **Mobile phase B** |
|---|---|---|
| **(min)** | % **(v/v)** | % **(v/v)** |
| **0** | 80 | 20 |
| **5** | 75 | 25 |
| **40** | 70 | 30 |
| **50** | 60 | 40 |
| **60** | 60 | 40 |
| **65** | 10 | 90 |
| **76** | 10 | 90 |

The eluate was collected in 7 fractions as indicated in Table 6.

**Table 6**

| **Fraction** | **Collection** | **Concentration** | **Fraction volume (l)** |
|---|---|---|---|
| | **(min)** | **(g/l)** | |
| **F1** | 28-32.5 | 0 | 1.42 |
| **F2** | 32.5-37.5 | 0 | 1.57 |
| **F3** | 37.5-40.5 | 0.1 | 0.94 |
| **F4** | 40.5-43 | 0.3 | 0.82 |
| **F5** | 43-47.2 | 0.1 | 1.32 |
| **F6** | 47.2-51.5 | 4.1 | 1.32 |
| **F7** | 51.5-57.5 | 0.8 | 1.87 |

The yield of the purification method was found to be 95%.

The fractions containing dalbavancin, resulting in specification, were concentrated and dialyzed against demineralized water (10 diavolumes), with a nanofiltration system with a molecular cut-off of 250 Da (HydroAirResearch) until a concentration of dalbavancin in the concentrate of 80 g/l was obtained. The concentrate was then brought to pH 2.6 +/-0.1 with 15% w/w aqueous hydrochloric acid (CarloErba). The selection of the eluate fractions to be concentrated is carried out by HPLC analysis by choosing the fractions with purity greater than or equal to 90% calculated on the sum of the areas of the peaks of the compounds that make up the antibiotic.

A total of 9 volumes of acetone (CarloErba) were then added to the dalbavancin solution in water at a temperature below 10°C. The solid was recovered after overnight incubation at 4°C by filtration on a porous septum, washed with 1.5 volumes of acetone (CarloErba) and dried under vacuum (50 ubar) at 15°C. The yield of the method was 84.0%.

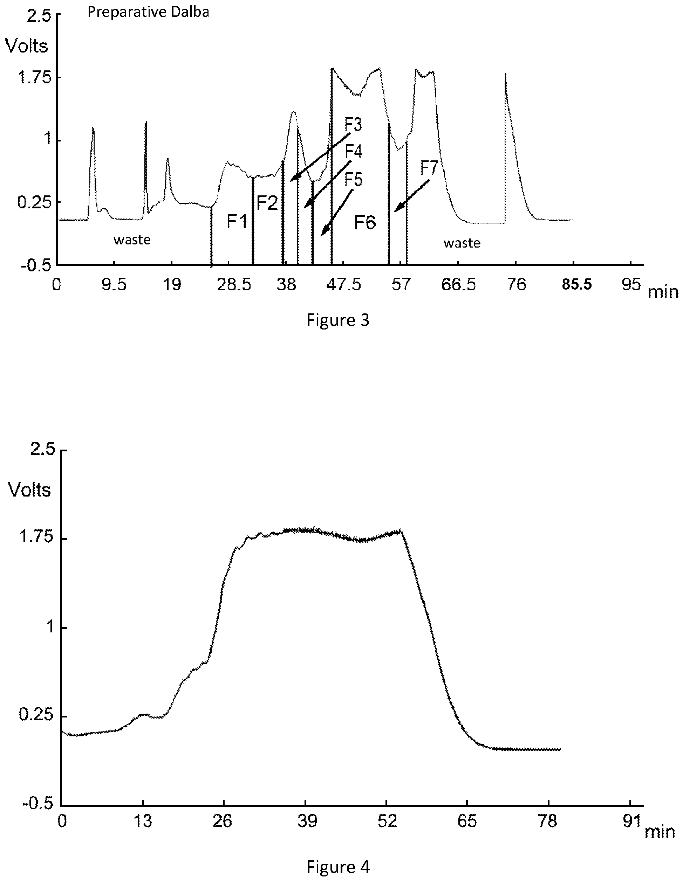

Figure 3 illustrates the chromatographic profile (tracing of the UV signal read at 280 nm) of the purification of dalbavancin obtained by preparative HPLC carried out by applying the conditions of example 2. The vertical lines indicate the boundary between one collection fraction and the next.

The chromatographic profile shows the high resolution between the various components of the mixture which, therefore, allows the almost complete removal of the impurities present.

### Example 4 - Purification of dalbavancin

On a preparative HPLC chromatographic column of 10 cm diameter and 60 cm packed height with stationary phase compound of octadecyl silyl derivatized silica (C18) with a particle size of 15 um (Phenomenex), conditioned with 0.025 M aqueous ammonium formate (CarloErba) pH 5.2 (mobile phase A), 680 ml of an aqueous solution of unrefined dalbavancin (active 7.3 g) was loaded, obtained as described in Example 2.

The column was then eluted at 320 ml/min with an eluent composition comprising acetonitrile (CarloErba) in an increasing concentration over the elution conduction time. This eluent composition was obtained following the mixing in time-varying quantitative ratios, as illustrated in Table 7, of a first solution (mobile phase A) comprising demineralized water with the addition of 0.025M aqueous ammonium formate (CarloErba) at pH 5.2 and a second solution (mobile phase B) of pure acetonitrile (CarloErba).

**Table 7**

| **Time (min)** | **Mobile phase A** (% **v/v)** | **Mobile phase B** % **(v/v)** |
|---|---|---|
| **0** | 80 | 20 |
| **60** | 65 | 35 |
| **90** | 60 | 40 |
| **110** | 50 | 50 |
| **120** | 10 | 90 |
| **140** | 10 | 90 |

The eluate was collected in 13 fractions as described in Table 8.

**Table 8**

| **Fraction** | **Collection (min)** | **Concentration (g/l)** | **Fraction volume (l)** |
|---|---|---|---|
| **F1** | 14.7-18 | 0 | 1.1 |
| **F2** | 43-51 | 0 | 2.6 |
| **F3** | 51-60 | 0 | 2.9 |
| **F4** | 60-69 | 0.1 | 2.9 |
| **F5** | 69-77 | 0.2 | 2.6 |
| **F6** | 77-83 | 0.1 | 1.9 |
| **F7** | 83-88 | 0.2 | 1.6 |
| **F8** | 88-95 | 0.4 | 2.2 |
| **F9** | 95-103 | 0.6 | 2.6 |
| **F10** | 103-112 | 0.8 | 2,9 |
| **F11** | 112-120 | 0.4 | 2.6 |
| **F12** | 120-127 | 0.2 | 2.2 |
| **F13** | 127-140 | 0 | 4.2 |

The yield of the purification method was found to be 95%.

Figure 4 illustrates the chromatographic profile (tracing of the UV signal read at 280 nm) of the purification of Dalbavancin obtained by preparative HPLC carried out by applying the conditions of example 4. The apparent absence of resolution is due to the high concentration of the mixture subjected to purification. However, it is possible to note that impurities accumulate at the beginning and end of the peak, thus allowing recovery of the pure product in the central part of the peak.

Compared with purification methods known in the art, the method described in this application allows a greater overall yield to be obtained. For example, the purification method carried out by polyamide stationary phase adsorption chromatography (as described, for example, in US 2004/0142883 A1) has a weight/weight yield of API dalbavancin between 25% and 33% weight/weight starting from the intermediate A40926 present in the initial fermentation broth. The method described herein has a yield by weight/weight of API dalbavancin, starting from A40926 initially present in the fermentation broth, greater than 35% weight/weight, with a yield increase that varies between 5% and 60%, on average 32%.

Furthermore, the purity of the product obtained with the described method is significantly higher than that of the product obtained by purification with adsorption chromatography on polyamide, for example, described in document US 2004/0142883 A1.

Table 9 below reports the results obtained by analyzing, by means of HPLC, a commercial sample of Dalbavancin (Xydalba, Durata Therapeutics) and a sample obtained by applying the purification method described in this description. It is possible to note that the total correlated substances are reduced by 50% and, above all, the impurity mannosyl aglycone (MAG), which originates by decomposition of the Dalbavancin, without antibiotic activity, is reduced by 88% by simultaneously increasing the active component.

**Table 9**

| **Parameter** | | **Method adsorption on polyamide** | **Method present descriptio n** |
|---|---|---|---|
| | | **[% area]** | **[% area]** |
| Complex distribution of the components | | | |
| | A₀+A₁ | 3.2 | 1.2 |
| | B₀ | 83.1 | 90.7 |
| | B₁+B₂ | 10.3 | 6.0 |
| Correlated substances | | 0.2 | <0.2 |
| | D₀+D₁ | <0.2 | <0.2 |
| | C₀+C₁ | 1.7 | 0.2 |
| | MAG | 0.6 | 0.5 |
| | IsoB₀ | 0.5 | 0.2 |
| | Trichloro | <0.2 | 0.3 |
| | DesAA-1 | 0.3 | 0.5 |
| | RRT 0.93 | <0.2 | <0.2 |
| | RRT 1.3 | <0.2 | 0.3 |
| | MA-A-1 / RRT1.43 | <0.2 | 0.2(rrt1.1 6) |
| Single substance not specified | | <0.2 | |
| Total of unspecified substances | | 3.3 | 0.2 |
| Total of correlated substances | | | 2.2 |

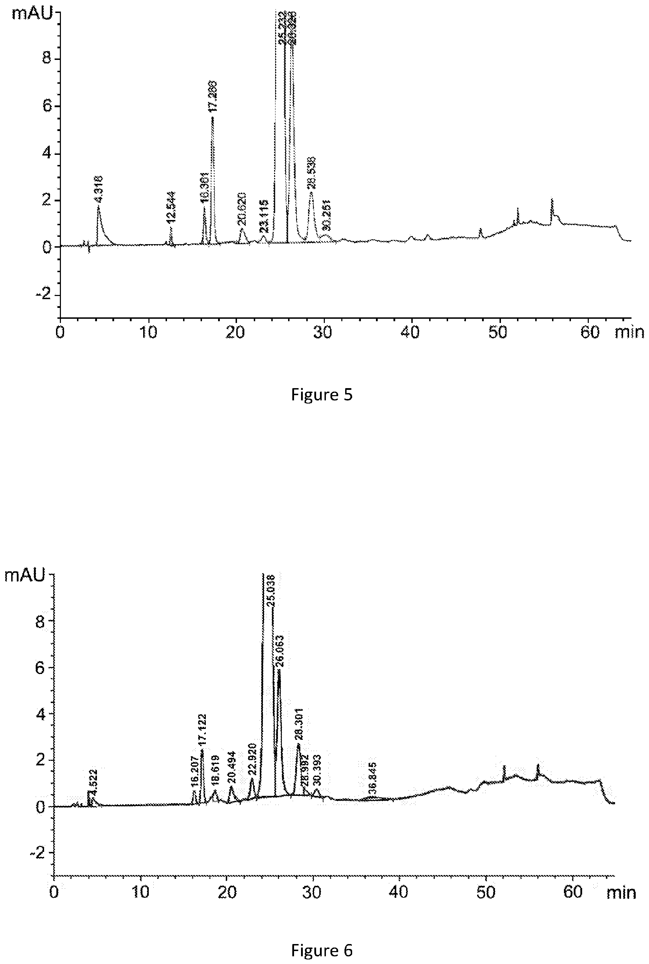

Figure 5 and Figure 6 illustrate HPLC chromatograms (tracing the UV signal read at 280 nm) obtained by analyzing, respectively, commercial dalbavancin (Xydalba, Durata Therapeutics) and dalbavancin obtained by applying the method described in this document. Both samples were injected at the same concentration. The chromatogram shown in Figure 6 (dalbavancin obtained by following the method described in the present description) shows the significant reduction of all the related impurities compared to the chromatogram shown in Figure 5 (commercial dalbavancin).

## Claims

1. Method for purifying at least one lipoglycopeptide antibiotic comprising the steps of:
i) dissolving said at least one lipoglycopeptide antibiotic in an aqueous solution to form a mixture,
ii) loading said mixture into a chromatographic column comprising a stationary phase, wherein said stationary phase comprises silica functionalized with organic pendants,
iii) eluting the mixture loaded in step ii) using an eluent composition comprising a water-soluble organic solvent obtaining eluate fractions,
iv) selecting the eluate fractions containing the at least one purified lipoglycopeptide antibiotic,
v) concentrating the eluate fractions selected in step iv) to obtain a concentrate,
vi) optionally dialyzing the concentrate obtained in step v),
vii) precipitating said concentrate, optionally dialyzed in step vi), with a precipitation solution comprising at least one organic solvent,
viii) drying the precipitate obtained in step vii),
wherein said concentration step v) is carried out by means of a membrane nanofiltration system with a molecular cut-off ranging from 100 Da to 1500 Da.

2. Method according to claim 1, wherein the at least one lipoglycopeptide antibiotic is selected in the group consisting of A40926, dalbavancin, teicoplanin, mideplanine (MDL-62873), and ramoplanin.

3. Method according to claim 1 or claim 2, wherein said silica functionalized with organic pendants is selected in the group consisting of octadecyl silyl derivatized silica, octyl silyl derivatized silica, exylphenyl silyl derivatized silica, and butyl silyl derivatized silica, preferably octadecyl silyl derivatized silica.

4. Method according to any one of the previous claims, wherein said silica functionalized with organic pendants has a particle size lower than 50 µm, preferably lower than 20 pm.

5. Method according to any of the previous claims, wherein the water-soluble organic solvent contained in the eluent composition of step iii) is selected in the group consisting of methanol, propanol, isopropanol, acetonitrile, acetone.

6. Method according to any of the previous claims, wherein the eluent composition further comprises at least one additional compound selected in the group consisting of ammonium formate, ammonium acetate, triethylamine, formic acid, acetic acid, trifluoroacetic acid, heptafluorobutyric acid, methanesulfonic acid.

7. Method according to any of the previous claims, wherein the eluent composition comprises said water-soluble organic solvent, preferably acetonitrile, in a concentration increasing over time, at least in one interval of conduction time of the elution step iii) or along the entire elution step iii).

8. Method according to any previous claim, wherein the elution step iii) is carried out at a pH between 5.0 and 7.0, preferably between 5.2 and 6.5.

9. Method according to any previous claim, wherein said stationary phase is subjected, before said step ii), to conditioning with a mobile phase which comprises at least one compound selected in the group consisting of ammonium formate, ammonium acetate or triethylamine salts with formic acid, acetic acid, trifluoroacetic acid.

10. Method according to claim 9, wherein said mobile phase for the conditioning of the stationary phase has a pH between 5.0 and 7.0.

11. Method according to any previous claim, wherein said precipitation solution comprises at least one organic solvent selected in the group consisting of acetonitrile, acetone, isopropanol, propanol, and ethanol, preferably acetone.

12. Method according to any previous claim, wherein the drying step is carried out by heating at a temperature ranging from 15°C to 30°C and, preferably, at a pressure lower than 150 mBar.

## Patentansprüche

1. Verfahren zur Reinigung von mindestens einem Lipoglykopeptid-Antibiotikum, welches folgende Schritte umfasst:
i) Auflösen des mindestens einen Lipoglykopeptid-Antibiotikums in einer wässrigen Lösung zum Bilden einer Mischung,
ii) Laden der Mischung in eine chromatografische Säule, welche eine stationäre Phase umfasst, wobei die stationäre Phase mit organischen Anhängseln funktionalisiertes Siliciumdioxid umfasst,
iii) Eluieren der in Schritt ii) geladenen Mischung mittels einer Eluentenzusammensetzung, welche ein wasserlösliches organisches Lösemittel umfasst, wodurch Eluatfraktionen erhalten werden,
iv) Auswählen der Eluatfraktionen, welche das mindestens eine gereinigte Lipoglykopeptid-Antibiotikum enthalten,
v) Konzentrieren der in Schritt iv) ausgewählten Eluatfraktionen zum Erhalten eines Konzentrates,
vi) wahlweise Dialysieren des in Schritt v) erhaltenen Konzentrates,
vii) Ausfällen des in Schritt vi) wahlweise dialysierten Konzentrates mit einer Fällungslösung, welche mindestens ein organisches Lösemittel umfasst,
viii) Trocknen des in Schritt vii) erhaltenen Fällungsproduktes,
wobei der Konzentrationsschritt v) mittels eines Membran-Nanofiltrationssystems mit einer molekularen Ausschlussgrenze im Bereich von 100 Da bis 1500 Da ausgeführt wird.

2. Verfahren nach Anspruch 1, wobei das mindestens eine Lipoglykopeptid-Antibiotikum ausgewählt ist aus der Gruppe bestehend aus A40926, Dalbavancin, Teicoplanin, Mideplanin (MDL-62873) und Ramoplanin.

3. Verfahren nach Anspruch 1 oder 2, wobei das mit organischen Anhängseln funktionalisierte Siliciumdioxid ausgewählt ist aus der Gruppe bestehend aus Octadecylsilyl-derivatisiertem Siliciumdioxid, Octylsilyl-derivatisiertem Siliciumdioxid, Exylphenylsilyl-derivatisiertem Siliciumdioxid und Butylsilyl-derivatisiertem Siliciumdioxid, vorzugsweise Octadecylsilyl-derivatisiertem Siliciumdioxid.

4. Verfahren nach einem der vorhergehenden Ansprüche, wobei das mit organischen Anhängseln funktionalisierte Siliciumdioxid eine Partikelgröße von weniger als 50 µm, vorzugsweise weniger als 20 µm, aufweist.

5. Verfahren nach einem der vorhergehenden Ansprüche, wobei das in der Eluentenzusammensetzung von Schritt iii) enthaltene wasserlösliche organische Lösemittel ausgewählt ist aus der Gruppe bestehend aus Methanol, Propanol, Isopropanol, Acetonitril und Aceton.

6. Verfahren nach einem der vorhergehenden Ansprüche, wobei die Eluentenzusammensetzung ferner mindestens eine zusätzliche Verbindung umfasst, die ausgewählt ist aus der Gruppe bestehend aus Ammoniumformat, Ammoniumacetat, Triethylamin, Ameisensäure, Essigsäure, Trifluoressigsäure, Heptafluorbuttersäure und Methansulfonsäure.

7. Verfahren nach einem der vorhergehenden Ansprüche, wobei die Eluentenzusammensetzung das wasserlösliche organische Lösemittel, vorzugsweise Acetonitril, in einer Konzentration umfasst, die mit der Zeit zunimmt, zumindest in einem Intervall der Durchführungszeit des Elutionsschrittes iii) oder während des gesamten Elutionsschrittes iii).

8. Verfahren nach einem der vorhergehenden Ansprüche, wobei der Elutionsschritt iii) bei einem pH-Wert zwischen 5,0 und 7,0, vorzugsweise zwischen 5,2 und 6,5, ausgeführt wird.

9. Verfahren nach einem der vorhergehenden Ansprüche, wobei die stationäre Phase vor Schritt ii) einer Konditionierung mit einer mobilen Phase unterzogen wird, welche mindestens eine Verbindung ausgewählt aus der Gruppe bestehend aus Ammoniumformat, Ammoniumacetat oder Triethylaminsalzen mit Ameisensäure, Essigsäure und Trifluoressigsäure umfasst.

10. Verfahren nach Anspruch 9, wobei die mobile Phase für das Konditionieren der stationären Phase einen pH-Wert zwischen 5,0 und 7,0 aufweist.

11. Verfahren nach einem der vorhergehenden Ansprüche, wobei die Fällungslösung mindestens ein organisches Lösemittel ausgewählt aus der Gruppe bestehend aus Acetonitril, Aceton, Isopropanol, Propanol und Ethanol, vorzugsweise Aceton, umfasst.

12. Verfahren nach einem der vorhergehenden Ansprüche, wobei der Trocknungsschritt durch Erhitzen bei einer Temperatur im Bereich von 15 °C bis 30 °C und, vorzugsweise, bei einem Druck von weniger als 150 mBar ausgeführt wird.

## Revendications

1. Procédé pour la purification d'au moins un antibiotique de lipoglycopeptide comprenant les étapes de :
i) dissolution dudit au moins un antibiotique de lipoglycopeptide dans une solution aqueuse pour former un mélange,
ii) charge dudit mélange dans une colonne de chromatographie comprenant une phase stationnaire, dans lequel ladite phase stationnaire comprend de la silice fonctionnalisée avec des groupes pendants organiques,
iii) élution du mélange chargé dans l'étape ii) en utilisant une composition d'éluant comprenant un solvant organique soluble dans l'eau obtenant des fractions éluées,
iv) choix des fractions éluées contenant le au moins un antibiotique de lipoglycopeptide purifié,
v) concentration des fractions éluées choisies dans l'étape iv) pour obtenir un concentré,
vi) éventuellement dialyse du concentré obtenu dans l'étape v),
vii) précipitation dudit concentré, éventuellement dialysé dans l'étape vi), avec une solution de précipitation comprenant au moins un solvant organique,
viii) séchage du précipité obtenu dans l'étape vii),
dans lequel ladite étape de concentration v) est réalisée au moyen d'un système de nanofiltration sur membrane avec un seuil de coupure moléculaire de 100 Da à 1 500 Da.

2. Procédé selon la revendication 1, dans lequel le au moins un antibiotique de lipoglycopeptide est choisi dans le groupe consistant en A40926, dalbavancine, téicoplanine, midéplanine, (MDL-62873), et ramoplanine.

3. Procédé selon la revendication 1 ou revendication 2, dans lequel ladite silice fonctionnalisée avec des groupes pendants organiques est choisie dans le groupe consistant en silice dérivatisée avec de l'octadécylsilyle, silice dérivatisée avec de l'octasilyle, silice dérivatisée avec de l'hexylphénylsilyle, et silice dérivatisée avec du butylsilyle, de préférence silice dérivatisée avec de l'octadécylsilyle.

4. Procédé selon l'une quelconque des revendications précédentes, dans lequel ladite silice fonctionnalisée avec des groupes pendants organiques présente une taille de particule inférieure à 50 µm, de préférence inférieure à 20 µm.

5. Procédé selon l'une quelconque des revendications précédentes, dans lequel le solvant organique soluble dans l'eau contenu dans la composition d'éluant de l'étape iii) est choisi dans le groupe consistant en méthanol, propanol, isopropanol, acétonitrile, acétone.

6. Procédé selon l'une quelconque des revendications précédentes, dans lequel la composition d'éluant comprend de plus au moins un composé supplémentaire choisi dans le groupe consistant en formate d'ammonium, acétate d'ammonium, triéthylamine, acide formique, acide acétique, acide trifluoroacétique, acide heptafluorobutyrique, acide méthanesulfonique.

7. Procédé selon l'une quelconque des revendications précédentes, dans lequel la composition d'éluant comprend ledit solvant organique soluble dans l'eau, de préférence l'acétonitrile, dans une concentration augmentant au cours du temps, au moins dans un intervalle de durée de réalisation de l'étape d'élution iii) ou tout au long de l'étape d'élution entière iii).

8. Procédé selon l'une quelconque des revendications précédentes, dans lequel l'étape d'élution iii) est réalisée à un pH de 5,0 à 7,0, de préférence de 5,2 à 6,5.

9. Procédé selon l'une quelconque des revendications précédentes, dans lequel ladite phase stationnaire est soumise, avant ladite étape ii), à un conditionnement avec une phase mobile qui comprend au moins un composé choisi dans le groupe consistant en formate d'ammonium, acétate d'ammonium ou sels de triéthylamine avec de l'acide formique, acide acétique, acide trifluoroacétique.

10. Procédé selon la revendication 9, dans lequel ladite phase mobile pour le conditionnement de la phase stationnaire présente un pH de 5,0 à 7,0.

11. Procédé selon l'une quelconque des revendications précédentes, dans lequel ladite solution de précipitation comprend au moins un solvant organique choisi dans le groupe consistant en acétonitrile, acétone, isopropanol, propanol, et éthanol, de préférence acétone.

12. Procédé selon l'une quelconque des revendications précédentes, dans lequel l'étape de séchage est réalisée par chauffage à une température de 15°C à 30°C et, de préférence, à une pression inférieure à 150 mBar.
